(19) Europäisches Patentamt
European Patent Office
Office européen des brevets

(11) EP 0 897 719 A1

(12) **EUROPEAN PATENT APPLICATION**

(43) Date of publication:
**24.02.1999 Bulletin 1999/08**

(51) Int Cl.⁶: **A61K 7/50**, A61K 7/48, A61K 7/02, A61K 7/00

(21) Application number: **98306081.5**

(22) Date of filing: **30.07.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **19.08.1997 EP 97306320**

(71) Applicants:
• **UNILEVER PLC**
**London EC4P 4BQ (GB)**
Designated Contracting States:
**GB IE CY**
• **UNILEVER N.V.**
**3013 AL Rotterdam (NL)**
Designated Contracting States:
**BE CH DE DK ES FI FR GR IT LI LU MC NL PT AT**

(72) Inventors:
• **Franklin, Kevin Ronald**
**Bebington, Wirral, Merseyside, L63 3JW (GB)**
• **Pereira, Mavis Claire**
**Bebington, Wirral, Merseyside, L63 3JW (GB)**

(74) Representative: **Mole, Peter Geoffrey**
**UNILEVER PLC**
**Patent Division**
**Colworth House**
**Sharnbrook**
**Bedford MK44 1LQ (GB)**

(54) **Topical cleansing composition**

(57) A topical cleansing composition comprising: a solid heat generating material that generates heat on contact with water, a substantially anhydrous carrier or diluent, and from 2% to 30% by weight of anionic surfactant.

## Description

The present invention relates to a topical cleansing compositions and in particular to a foaming topical cleansing composition.

Aqueous foaming cleansing compositions for use as, for example, facial washes, hand washes or shower gels are very well known. Some non aqueous cleansing compositions are also known.

For example, WO 96/02230 discloses an aqueous liquid cleansing moisturising composition.

JP 08/059 455 discloses non foaming, non aqueous compositions comprising nonionic surfactant and self-heating components.

The present inventors have realised that the cleansing effect and sensation when using a topical cleansing composition may be increased if the composition produces heat when it is used. It is suggested to use a composition which is non-aqueous and which generates heat upon mixing with water.

WO 93/08793 provides a topical cleansing composition, mainly for use as a face mask, containing 3-60% of absorbent materials and 0.5-80% by weight of molecular sieve. Upon mixing with water, the molecular sieve absorbs water and produces heat of hydration. This heat opens the pores of the skin to enable soil, grease or oil to be more readily released so that it is more easily absorbed by the absorbent material. Small quantities of cosmetically acceptable surfactants may be included. Polyethoxylated glyceryl laurate or polyethoxylated sorbitol are mentioned. However, this composition has not proved suitable for use as a foaming topical cleansing composition.

JP 08/059,455 discloses nonionic based face-cleansing compositions.

The present inventors have discovered that a non-aqueous topical cleansing composition containing a material which generates heat upon hydration and contains certain quantities of anionic surfactant can provide a self-heating foaming topical cleansing composition.

Accordingly, in a first aspect the present invention provides a topical cleansing composition comprising:

a solid heat generating material that generates heat on contact with water, a substantially anhydrous carrier or diluent, and

from 2 to 30% by weight of anionic surfactant.

Compositions according to the present invention generate a noticeable sensation of heat when first applied to the skin. This is believed to be due to absorption of water from the skin. Upon further working of the composition and addition of water, further heat and foam is produced, which can last for comparatively long periods.

It is especially preferred that the topical cleansing composition is a foaming composition.

The heat generating material in compositions according to the invention is preferably an aluminosilicate, such as an amorphous or crystalline aluminosilicate. It is particularly preferred that the aluminosilicate is zeolite, most preferably anhydrous zeolite. The zeolite may be present in an amount of 5-60% by weight, preferably 10-50 % by weight based upon the total composition.

Small pore (desiccant) silica or activated hydrotalcite may also be used, as at least part of the heat generating material. Silica is also a preferred ingredient as it provides the benefit of assisting in stabilizing the composition.

Where zeolite is used, it may be any suitable form of zeolite. For example, zeolite A, zeolite X such as provided by Fluka or Union Carbide, or zeolite MAP as described in EP-A-384,070 are preferably used. The ion exchange form of the zeolite can be varied to control the heat of hydration, rate of hydration and alkalinity. Typical cations in subject zeolites include Na, K, Ca, Zn, Mg, Li, Cu and combinations of these ions. In terms of pH, the zeolite may be slightly acid, slightly alkaline or neutral.

Mixtures of different types of zeolite materials may also be used.

The zeolite or other solid heat generating material is preferably substantially anhydrous. The zeolite may be rendered anhydrous by heating in a furnace at high temperature for a suitable time, preferably followed by cooling in the presence of a desiccant or by other suitable processes such as freeze drying etc. For example, the material may be heated at a temperature in the region 200-500°C, preferably 400°C or above for a period of at least 24 hours, preferably 48 hours. A desiccant such as phosphorous pentoxide may be used.

Preferably, the material which generates heat is present in an amount suitable to generate a temperature rise which can be readily appreciated by the user. For example, a temperature increase in the range 5-30°C, preferably 5-15°C over a period of 0.1-5 minutes, preferably 0.5-2 minutes may be obtained.

The heat generating material is preferably present at a level of at least 5% by weight, preferably at least 10% by weight of the total composition. It is preferred that the heat generating material is present at a level of between 5-60% by weight, preferably 10-50% by weight of the composition.

The solid heat generating material is preferably carried in a dermatologically acceptable carrier or diluent.

The carrier or diluent should be substantially anhydrous. The term "anhydrous" is used herein to cover compositions where the temperature increase upon hydration is not substantially less than the heat increase on hydration of material containing 0% of water.

Preferably, the water content is less than 1%, preferably less than 0.5%, most preferably less than 0.05% by weight.

The carrier functions to suspend the solid heat producing material to provide a composition which is easy to apply to the skin. The carrier or diluent may also provide texture to the skin cleaning composition. The diluent may be any suitable dermatologically compatable non-aqueous liquid. Suitable diluents include alcohols or glycerol. Preferably the carrier or diluent comprises a water miscible substantially anhydrous liquid.

The diluent or carrier is typically present in amounts of between 5% and 50% by weight, based on the total weight of the composition, preferably 10 to 40%, most preferably 15 to 35%.

The diluent may contain a structurant. A structurant is in this case a material which will increase the zero shear rate viscosity. For example, swelling clay such as laponite, fatty acids and derivatives thereof such as fatty acid monoglyceride polyglycol ether, cross-linked polyacrylates such as Carbopol (Trademark of Goodrich), acrylates and copolymers thereof, polyvinylpyrrolidone and copolymers thereof, polyethyleneimines and salts such as sodium chloride and ammonium sulphate, sucrose esters, gellants and mixtures thereof may be used.

The diluent may also contain a thickening agent, i.e. a material which maintains the viscosity of the composition as the shear rate is increased. Suitable materials include Carbopol (Trademark of Goodrich), fatty acids and derivatives thereof, in particular fatty acid monoglyceride polyglycol ether, natural gums including alginates and guar and xanthan gum, and polysaccharide derivatives such as carboxy methyl cellulose and hydroxy propyl guar, propylene glycols, propylene glycol oleates, salts such as sodium chloride and ammonium sulphate, glyceryl tallowates and mixtures thereof.

The compositions of the present invention comprise from 2 to 30% by weight of anionic surfactant, preferably 2 to 20% by weight, most preferably 3 to 13% by weight.

The compositions contain anionic surfactant as the predominant surfactant although other surfactant types may be present if anhydrous. Preferred are mixtures of anionic and nonionic surfactant. The surfactant may comprise a compatable mixture of anionic with zwitterionic, amphoteric or cationic surfactants if anhydrous.

Indeed, it is a highly preferred embodiment of the invention that the composition comprises a mixture of anionic and nonionic surfactant, as the presence of a nonionic surfactant can assist in boosting the mildness of the topical composition. When a nonionic surfactant is used in the composition, it is preferably present at a level of 1-10%, more preferably 2-8% by weight of the composition.

The surfactant is preferably a mild surfactant, i.e. a surfactant which does not damage the stratum corneum, the outer layer of the skin.

Other less mild surfactant may be present, although it is preferred that the amount of other surfactant present will not be such as to affect the mildness or foaming of the composition.

A preferred type of anionic surfactant is fatty acyl isethionates of formula:

$$RCO_2CH_2CH_2SO_3M$$

where R is an alkyl or alkenyl group of 7 to 21 carbon atoms and M is a solubilising cation such as sodium, potassium, ammonium or substituted ammonium. Preferably at least three quarters of the RCO groups have 12 to 18 carbon atoms and may be derived from coconut, palm or coconut/palm blends.

Other preferred anionic surfactants are sarcosinates of formula:

$$R^5CON(CH_3)CH_3CO_2M,$$

wherein $R^5$ ranges from $C_8$ - $C_{20}$ alkyl, preferably $C_{12}$- $C_{15}$ alkyl and M is a solubilising cation.

Sulphosuccinates may also be present in the compositions of the present invention, for example, monoalkyl sulphosuccinates having the formula:

$$R^5O_2CCH_2CH(SO_3M)CO_2M;$$

and amido-MEA sulphosuccinates of the formula:

$$R^5 COHNCH_2CH_2O_2CCH_2CH(SO_3M)CO_2M;$$

wherein $R^5$ ranges from $C_8$-$C_{20}$ alkyl, preferably $C_{12}$-$C_{15}$ alkyl, and M is a solubilising cation.

Taurates may also be present in the compositions of the present invention, for example, those generally identified by the formula:

$$R^5 CONR^6 CH_2CH_2SO_3M,$$

wherein $R^5$ ranges from $C_8$ - $C_{20}$ alkyl, preferably $C_{12}$- $C_{15}$ alkyl, $R^6$ ranges from $C_1$ - $C_4$ alkyl and M is a solubilising cation.

Other possible anionic surfactants which may be incorporated in the compositions of the present invention include alkyl glyceryl ether sulphate, sulphoacetates, alkyl phosphate, alkyl phosphate esters and acyl lactates, alkyl glutamates and mixtures thereof.

Harsh surfactants such as primary alkane sulphonate or alkyl benzene sulphonate will generally be avoided unless incorporated in small amounts so as not to adversely affect the mildness of the present compositions. Particularly preferred anionic surfactants include sodium lauryl sarcosinate and sodium cocyl isethionate

Suitable nonionic surfactants which may be used include alkyl polysaccharides, lactobionamides, ethyleneglycol esters, glycerol monoethers, polyhydroxyamides (glucamide), primary and secondary alcohol ethoxylates, especially the $C_{8-20}$ aliphatic alcohols ethoxylated with an average of from 1 to 20 moles of ethylene oxide per mole of alcohol.

Preferred species of nonionic surfactant for use in compositions according to the invention are alcohol ethoxylate surfactants, and polyoxyethylene surfactants.

Particularly preferred ones include polyoxyethylene 4 lauryl ether and polyoxyethylene sorbitol.

It is possible to include any surfactant or co-surfactant which exhibits skin mildness benefits and which is available in substantially anhydrous form or compatible mixtures thereof.

It is particularly preferred that the surfactant system should give good foaming properties. The surfactant system must of course be substantially anhydrous. Preferably, the surfactant system comprises good foaming surfactants such as fatty acyl isethionate, taurate, sarcosinate, betaines, amidobetaines or sulphobetaines.

The total amount of surfactant present in the compositions is preferably in the range 2 to 30% by weight, most preferably 2 to 20%. The surfactant is incorporated into the compositions in a substantially anhydrous phase.

Inert solids such as titanium dioxide, abrasive particles or non-abrasive particles, e.g. powdered silica (such as Aerosil ex Degussa) may optionally be included according to the desired properties of the composition.

The composition of the invention may also include other known additives provided that they are anhydrous. For example, benefit agents as listed in WO 96/02224 may be included in the present invention, such as moisturising agents.

A preferred optional component is an oil, which is preferably present at a level of 0.1-3% by weight of the composition. It is however preferably not present at a level of higher than 3%, since at levels above this there may be a tendency for the composition to exhibit stickiness on application to the skin.

The composition of the invention may be used in shampoo, hair conditioner, or other such personal care products, but is preferably a skin cleansing composition.

The composition according to the present invention may also include perfume or opacifier. For example, 0.01-1, preferably about 0.5% by weight of perfume may be included.

The composition of the present invention is preferably in a liquid, paste, gel, thick cream or other form suitable for application to skin.

Typically the viscosity of the paste compositions of the present inventions are within the range 150,000 cp to 250,000 cp, for example 175,000 cp to 220,000 cp as measured on a Brookfield viscometer (spindle D, speed 5).

The present invention may be packaged in a container designed to prevent deactivation of the composition, by ingress of water after the container is opened. For example, the composition may be contained in containers or sachets each providing a unit dose which is used up at once, Alternatively, the composition of the present invention may be a spreadable composition contained in a flexible tube with an opening for extrusion of the composition. Preferably, the opening is relatively small (for example, it may have an area of 0.1 $cm^2$ - 1 $cm^2$ preferably around 0.2-0.7$cm^2$.

It has been found that the kinetics of heat production upon hydration are particularly important.

It has been observed that the temperature of the composition of the present invention upon hydration increases with time up to a peak and then decreases steadily. The kinetics of heat production affect both the height of the temperature peak and the time after hydration at which it occurs.

It is particularly preferred that the peak occurs 0.5-2 minutes after contact with water. The hydration kinetics can be altered by using different forms of zeolite. A preferred example, zeolite Na-A, has a substantially faster rate of heat production than zeolite Na MAP.

The compositions of the present invention find particular application in facial cleansing, and also, for make-up removal. Deep cleansing compositions such as face packs are also included within the scope of the present invention. The present invention will be further described by way of example only with reference to the following non-limiting examples. Other modifications within the scope of the present invention will be obvious to the skilled person.

EXAMPLE 1 - Skin Cleansing

A foaming topical cleansing composition A according to the present invention and having the following formulation was produced.

Table 1;

| Composition A | | |
| --- | --- | --- |
| **Ingredient Chemical Name** | **Trade name & Supplier** | **%ww Active Ingredient** |
| Zeolite Na A | (Molecular Sieve Union Carbide Type 4A) ex Fluka | 37.00 |
| Kaolin | Heavy Kaolin BP ex Goonvean & Rostowrack | 1.00 |
| Titanium dioxide | R - TC4ex Tioxide | 1.00 |
| Butane 1,3 diol | 1,3 butylene glycol ex Huls | 5.00 |
| Polyethylene-glycol 400 | PEG-400ex J T Baker | 20.50 |
| Glycerol | Glycerine ex J T Baker | 15.00 |
| Silica (amorphous) | Aerosil 200ex Degussa | 2.00 |
| Polyoxyethylene 30 sorbital | Atlas G2330ex ICI | 5.00 |
| Sodium carboxymethyl cellulose | ex Aqualon | 1.00 |
| Sweet Almond oil | ex Jan Dekker | 0.50 |
| Sodium cocoyl isethionate | Jordapon CI/Elfan AT 84ex PPG/Mazer or Akzo | 8.00 |
| Sodium lauroyl sarcosinate | Hamposyl L-95ex Hampshire | 4.00 |
| | | 100.00 |

The zeolite was dehydrated by placing in an oven at 400°C for 48 hours followed by placing the zeolite in a dessicator containing phosphorous pentoxide dessicant whilst cooling.

The sodium cocoyl isethionate, sodium lauroyl sarcosinate, PEG-400, butane 1,3 diol and a glycerol were heated to 80°C to solubilize the surfactant in the diluent. The remaining components were then intimately mixed together and homogenised.

The composition of example 1 was tested as follows:-

(i) STORAGE TEST
The composition of the invention was placed in a sealed jar and placed in a incubator/oven maintained at 45°C. The composition was stable for 6 months. No degradation was observed in the sample during this time. A standard requirement is stability for at least 3 months at this temperature.

(ii) HEATING EFFECT
To measure the increase in temperature upon hydration, a sample of the composition was mixed with water and the temperature increase after hydration measured using a thermocouple. Figure 1 shows the heating profile obtained.

## Figure 1

A temperature increase of approximately 9°C was obtained within one minute, followed by a slow decrease in temperature of the composition.

(iii) FOAMING TEST
The generation of foam was assessed by the following method. 0.5 grams of skin cleansing composition was weighed out. The weighed sample of the composition was poured onto the hands at a sink and rubbed into the hands. The sensation before the addition of water and after the addition of water was assessed subjectively. As a comparison, Composition B was also tested.

Composition A corresponds to corresponds to example 1 above.
Composition B is substantially the same as example 1, except that the quantities of glycerol, sodium cocoyl isethionate and sodium lauroyl sarcosinate were as follows:

Glycerol - 21%
sodium cocoyl isethionate - 4%
sodium lauroyl sarcosinate - 2%.

EXAMPLE 2: Comparison Of Composition A and Composition B of Example 1.

(i) Skin Cleansing when washing with water

Composition A of Example 1 had a pasty feel on rub-in. A strong heat signal to the user was experienced as water was added. On addition of more water, a fine-textured foam was produced which was mild and creamy in nature. This was rinsed away easily, leaving no residual feel.
Composition B of Example 1 was by comparison, a slightly less pasty, more easily spreading composition. On addition of water, a comparable feeling of heat was produced. On further addition of water, a very small amount of foam was produced. The bubbles broke down quickly to form a milky liquor which rinsed away easily.
Further, the sensation of heat appeared to be more prolonged with lower quantities of surfactant (the subjectively tested level of heat lasted a few seconds longer). This may be due to the increase in the level of glycerol in the second composition.

(ii) Make-up Removal

Evaluation of the make-up removal properties of the composition was performed on a panel of nine volunteer laboratory personnel. The test was repeated using a commercially available conventional cold cream deep cleanser (Composition C) .

A plastic template was used to define three, 3cm by 2cm rectangular areas on the panellist's volar forearm. The colour of the skin within each site was recorded using a Minolta Chroma Meter CR-100 to give a set of baseline readings (A). The three-dimensional colour coordinate system L*a*b* was used and six measurements were made over each area to give an average value.

The designated areas were then coated with the test make-up (lipstick), which was applied in a standard way to ensure that approximately equal weights of make-up were transferred (average 7mg per site). After drying, a second colour measurement was made (reading B).

A standard amount of test product (0.2g) was applied to the skin, a different product being used on each make-up patch. The cleanser was rubbed into the make-up for 40 rubs (20s), using light finger pressure (gloved hand), after which the mixture was immediately rinsed off with running tap water at a fixed flow rate and temperature (6 litres/min, 35°C). The arm was held under the tap for 20s or for sufficient time to remove the cleanser.

After all three patches had been similarly treated, the arm was dried with a hair drier and final colour measurements were made (reading C).

The percentage removal was calculated as:

$$\frac{B-C}{B-A} \times 100$$

where $B - A = \sqrt{(LB-LA)^2 + (aB-aA)^2 + (bB-bA)^2}$

The experimental data were recorded from the memory of the Chroma Meter into a PC and processed using a programme for statistical analysis, which takes into account variability arising from different products, panellists and sites of application.

| RESULTS | | |
|---|---|---|
| **Product** | **% Make-up Removal** | **Standard Error (+/-)** |
| Example A | 75.19 | 1.09 |
| Example C | 58.37 | 1.08 |

Composition A of Example 1 exhibited good make-up removal when tested as above.

From the above results it can be demonstrated that make-up removal is substantially improved by the use of the compositions of the present invention which exhibit excellent make-up removal, for example, when compared to conventional cold creams.

**Claims**

1. A topical cleansing composition comprising:

   a solid heat generating material that generates heat on contact with water,
   a substantially anhydrous carrier or diluent and from 2% to 30% by weight of anionic surfactant.

2. The topical cleansing composition of claim 1, wherein the composition is a foaming composition.

3. The topical cleansing composition of claim 1 or claim 2, wherein the composition additonally comprises a nonionic surfactant.

4. The topical cleansing composition of claim 3, wherein the nonionic surfactant is present at a level of 1-10% by weight of the composition.

5. The topical cleansing composition of claim 3 or claim 4, wherein the level of anionic and nonionic surfactant in the composition is at least 6% by weight.

6. The topical cleansing composition of any one of the preceding claims, wherein the solid heat generating material is a zeolite.

7. The topical cleansing composition of claim 6, wherein the zeolite is substantially anhydrous.

8. The topical cleansing composition of either claim 6 or claim 7, wherein the zeolite is zeolite A.

9. The topical cleansing composition of either of claims 6 or 7, wherein the zeolite cation is selected from Na, K, Ca, Zn, Mg, Li, Cu or mixtures thereof.

10. The topical cleansing composition of any preceding claim, wherein the composition comprises less than 1% by weight of water.

11. The topical cleansing composition of any one of the preceding claims, wherein the anhydrous carrier or diluent comprises a water miscible substantially anhydrous liquid.

12. The topical cleansing composition of claim 11, wherein the substantially anhydrous liquid is present in an amount of from 10 to 40% by weight.

13. The topical cleansing composition of any one of the preceding claims, wherein the anionic surfactant comprises isethionates and/or sarcosinates.

14. The topical cleansing composition according to any preceding claim, wherein the composition comprises 6-30% by weight total surfactant.

15. The topical cleansing composition of any one of the preceding claims, which is a skin wash composition.

16. Use of a composition of any one of claims 1 to 15 as a make-up removal composition.

**European Patent Office**

## EUROPEAN SEARCH REPORT

Application Number

EP 98 30 6081

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 3 250 680 A (MENKART ET AL.) 10 May 1966 * column 1, line 1 - column 2, line 41; claims 1-7; examples 9,4 * | 1-16 | A61K7/50 A61K7/48 A61K7/02 A61K7/00 |
| X | US 4 187 287 A (SCHREIBER ET AL.) 5 February 1980 * the whole document * | 1-14 | |
| A,D | WO 93 08793 A (THE BOOTS COMPANY) 13 May 1993 * the whole document * | 1-16 | |
| A | GB 1 357 000 A (BRITISH-AMERICAN TOBACCO COMPANY) 19 June 1974 * the whole document * | 1-16 | |
| A | EP 0 187 912 A (PQ CORPORATION) 23 July 1986 * the whole document * | 1-16 | TECHNICAL FIELDS SEARCHED (Int.Cl.6) A61K |

The present search report has been drawn up for all claims

| Place of search | Date of completion of the search | Examiner |
|---|---|---|
| THE HAGUE | 11 December 1998 | Fischer, J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)